(19) Europäisches Patentamt
European Patent Office
Office européen des brevets

(11) **EP 1 406 701 B1**

(12) **EUROPÄISCHE PATENTSCHRIFT**

(45) Veröffentlichungstag und Bekanntmachung des Hinweises auf die Patenterteilung:
**31.08.2005   Patentblatt 2005/35**

(21) Anmeldenummer: **02748820.4**

(22) Anmeldetag: **26.06.2002**

(51) Int Cl.⁷: **A62B 27/00**, G09B 23/28, A61B 5/00, A61M 15/00, A61M 16/00, G01F 1/00, G01F 3/00, G01F 7/00, G01F 11/00, G01F 13/00, A61M 5/172

(86) Internationale Anmeldenummer:
**PCT/EP2002/007040**

(87) Internationale Veröffentlichungsnummer:
**WO 2003/004101 (16.01.2003 Gazette 2003/03)**

(54) **STEUEREINHEIT ZUR FLUSSREGULIERUNG**

CONTROL UNIT FOR FLOW REGULATION

UNITE DE COMMANDE POUR REGULATION DE FLUX

(84) Benannte Vertragsstaaten:
**AT BE CH CY DE DK ES FI FR GB GR IE IT LI LU MC NL PT SE TR**

(30) Priorität: **02.07.2001  DE 10131516**

(43) Veröffentlichungstag der Anmeldung:
**14.04.2004   Patentblatt 2004/16**

(73) Patentinhaber: **Boehringer Ingelheim Pharma GmbH & Co.KG
55216 Ingelheim am Rhein (DE)**

(72) Erfinder:
• **WOLF, Harald
55452 Rümmelsheim-Burg Layen (DE)**
• **FACHINGER, Andreas
55270 Ober-Olm (DE)**
• **MEMMESHEIMER, Holger
55437 Ockenheim (DE)**

(56) Entgegenhaltungen:
DE-A- 19 714 684          DE-A- 19 720 701
FR-A- 2 800 288           GB-A- 2 351 155
US-A- 4 730 500           US-A- 5 669 877
US-A- 5 887 586

• **BELFORTE G ET AL: "A tester for artificial respirators" MEASUREMENT, INSTITUTE OF MEASUREMENT AND CONTROL. LONDON, GB, Bd. 27, Nr. 4, Juni 2000 (2000-06), Seiten 241-250, XP004197438 ISSN: 0263-2241**

**Beschreibung**

Die vorliegende Erfindung betrifft eine Steuereinheit zur Flussregulierung, die in Verbindung mit einem Sammelrohr zur Ermittlung der Gleichförmigkeit einer aus einem Pulverinhalator ausgebrachten Medikamentendosis bzw. in Verbindung mit einem (Kaskaden-) Impaktor zur Ermittlung der Verteilung des aerodynamischen Feinanteils in dieser ausgebrachten Dosis eingesetzt wird. Die Erfindung betrifft außerdem Mess- und Dosis-Sammelrohre zur Verwendung in Verbindung mit der Steuereinheit, sowie ein Verfahren zur Ermittlung der genannten Parameter unter Einsatz der Steuereinheit und der Sammelrohre.

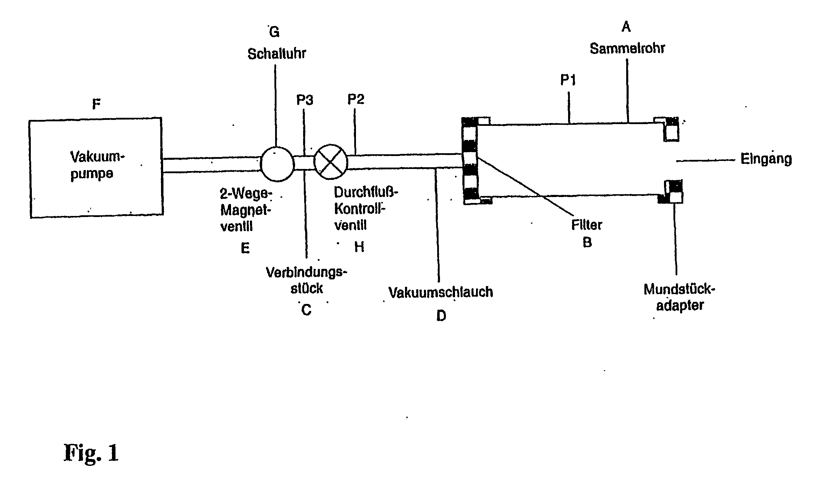

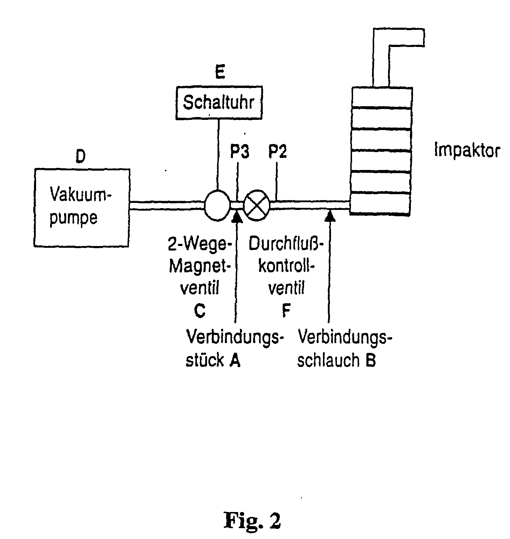

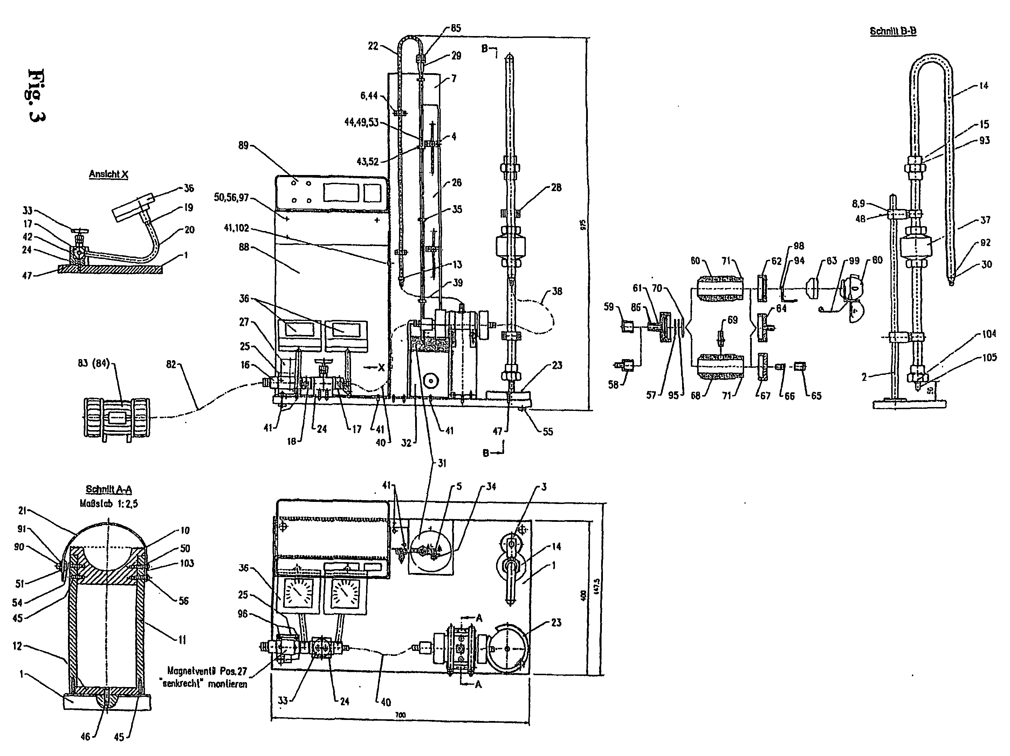

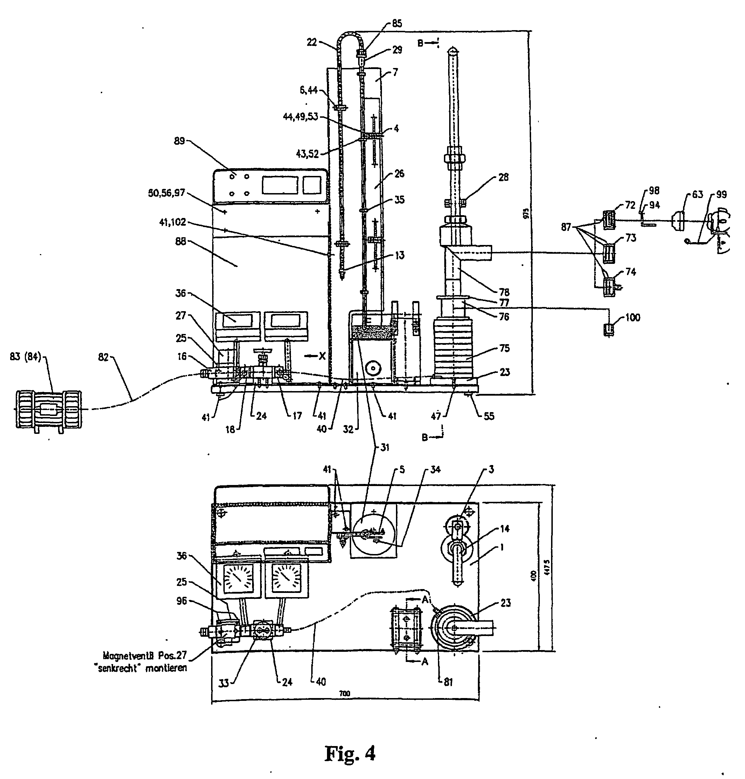

Für die Wirksamkeit eines Medikaments, das als Inhalationspulver aus einem Inhalator (z.B. einem Einzeldosis-Inhalator wie dem "HandiHaler®" oder dem "Inhalator Ingelheim"oder einem Mehrdosis-Inhalator) freigesetzt wird, ist die abgegebene Wirkstoffmenge und deren aerodynamische Partikelgrößenverteilung entscheidend. Diese Prüfparameter dienen der Beurteilung der Qualität des Arzneimittels und sind im Rahmen von Stabilitätsprüfungen zu ermitteln. Die Verfahren zur Bestimmung dieser Parameter sind in der US-Pharmakopoe 24, S. 1896 ff. (nachfolgend: USP) und der Europäischen Pharmakopoe, 3. Auflage, Nachtrag 2000, S. 1540 ff. und S. 101 ff. (nachfolgend: EP) normiert. Die Pharmakopöen enthalten Vorgaben für die bei der Bestimmung einzuhaltenden physikalischen Parameter (z.B. die Größe des zur Ausbringung der Medikamentendosis anzulegenden Unterdrucks) und den grundlegenden Aufbau der für diese Bestimmungen einzusetzenden Vorrichtungen. Die EP/USP enthält z.B. die in Fig. 1 und Fig. 2 gezeigten Schemazeichnungen.

Das Prinzip der Bestimmung der abgegebenen Wirkstoffmenge bzw. der aerodynamischen Partikelgrößenverteilung beruht darauf, dass bei einem definiert angelegten Volumenstrom der Wirkstoff aus einem Inhalator ausgebracht wird. Die abgegebene Pulvermenge wird in einer speziellen Vorrichtung (Dosis-Sammelrohr bzw. Kaskadenimpaktor) vollständig aufgefangen und anschließend der quantitativen Analyse zugeführt.

Wie in Fig. 1 und Fig. 2 zu sehen ist, besteht die in der EP/USP schematisch gezeigte Vorrichtung aus einer Saugeinheit in Form einer Vakuumpumpe, einem durch eine Schaltuhr steuerbaren 2-Wege-Magnetventil, einem Durchflusskontrollventil sowie einem Sammelrohr bzw. einem Impaktor. Vakuumpumpe und 2-Wege-Magnetventil, 2-Wege-Magnetventil und Durchflusskontrollventil sowie Durchflusskontrollventil und Sammelrohr bzw. Kaskadenimpaktor sind miteinander verbunden. P1, P2 und P3 bezeichnen Positionen, an denen Druckmessgeräte angebracht werden können.

Bei der Durchführung der Bestimmung wird durch den Sog der Vakuumpumpe im Sammelrohr bzw. im Kaskadenimpaktor ein definierter Unterdruck erzeugt. Durch den Unterdruck wird der Inhalator von einem bestimmten Volumenfluss durchströmt, wodurch Pulverpartikel aus dem Inhalator in das Dosis-Sammelrohr bzw. den Impaktor ausgebracht werden. Die darin aufgefangene Wirkstoffmenge wird anschließend mit einer geeigneten Methode, beispielsweise HPLC, quantifiziert.

Um die Messungen unter standardisierten und reproduzierbaren Bedingungen durchführen zu können, muss vor allem ein definierter Volumenfluss, mit dem die Ausbringung der Medikamentendosis aus dem Inhalator erfolgt, gewährleistet werden. Die EP/USP geben z.B einen Volumenfluss von 4 Litern vor ("gefordertes Saugvolumen"). Um das geforderte Saugvolumen zu erreichen, wird ein vorgegebener Unterdruck von 4 kPa an die Vorrichtung angelegt. Die Grösse des Volumenstroms hängt stark von der Konstruktionsweise der Apparatur und deren Bestandteile ab, die sich in einem bestimmten Strömungswiderstand niederschlägt. Aus dem Volumenstrom lässt sich dann errechnen, wie lange der definierte Unterdruck anliegen muss (z.B. das Magnetventil in Fig. 1 oder Fig. 2 geöffnet sein muss), damit bei einem Messvorgang mit angeschlossenem Inhalator ein Volumenfluss von 4l erreicht wird ("Saugzeit"; Details s. unten). Damit hängt die Genauigkeit der Messung u.a. von der genauen Kontrolle der Saugzeit und der genauen Bestimmung des Volumenflusses ab. Bezüglich des letztgenannten Parameters macht die USP/EP folgende Vorgaben: In der USP werden für die Ausbringung ± 5% für den Volumenfluss von 4l, den Volumenstrom (Q) und die Zeit angegeben. Bei der aerodynamischen Teilchengrößenverteilung (PSD) werden nur ± 5% für die Zeit gefordert. Die EP lässt bei der Ausbringung bei einem Volumenstrom von max. 100l eine Toleranz von ± 5% zu. Bei der PSD werden ± 5% für den Volumenstrom (Q) und die Zeit verlangt.

Geräte, die sich für Messungen nach den Vorgaben der EP/USP eignen, waren zum Zeitpunkt der Erfindung auf dem Markt nicht erhältlich. Versuche, auf der Basis der in der EP/USP enthaltenen schematischen Zeichnungen entsprechende Messvorrichtungen in eigener Fertigung zu realisieren, blieben zunächst erfolglos. Insbesondere zeigte sich, dass mit einem wie in Fig. 1 und Fig. 2 gezeigtem Aufbau der Vorrichtungen in keinem Fall ein hinreichend konstanter Volumenstrom gewährleistet werden kann (interner Stand der Technik).

Darüber hinaus zeigte sich bei den genannten Versuchen, dass immer dann, wenn die probeweise in das Sammelrohr ausgebrachten Pulvermengen gering waren (z.B. im Bereich von 4 - 5 μg), bei der anschließenden quantitativen Bestimmung des Sammelrohrinhalts erhebliche Schwankungen auftraten. Dies konnte darauf zurückgeführt werden, dass Pulver in den in Fig. 1 mit P 1 bezeichneten Bereich (Anschlussstelle für ein Druckmessgerät) eingedrungen war und daher bei der quantitativen Bestimmung des Sammelrohrinhalts nicht erfasst wurde.

Es ist daher eine Aufgabe der Erfindung eine Vorrichtung bereit zu stellen, mit der die nach EP/USP vorgeschriebenen Messungen zur Ermittlung der Gleichförmigkeit einer aus einem Pulverinhalator ausgebrachten Medikamentendosis bzw. zur Ermittlung der Verteilung des aerodynamischen Feinanteils dieser Dosis mit der erforderlichen Genauigkeit durchgeführt werden können.

Eine weitere Aufgabe der Erfindung ist es, ein Sammelrohr und ein Verfahren unter Verwendung eines solchen Sammelrohres bereitzustellen, durch die der oben genannte Nachteil, nämlich eine Verfälschung des Messergebnisses durch Pulvermengen, die sich im Anschlussbereich P 1 verfangen, vermieden werden kann.

Die Aufgaben werden gelöst durch eine Steuereinheit gemäß den Ansprüchen 1 - 5 sowie Sammelrohren gemäß den Ansprüchen 6 oder 7 zur Verwendung in einer Vorrichtung gemäß der EP/USP sowie durch ein Verfahren gemäß dem Anspruch 9.

Die Steuereinheit zur Flussregulierung basiert auf dem in Fig. 1 und Fig. 2 gezeigten grundlegenden, durch die EP/USP vorgegebenen Aufbau und enthält damit ein durch eine Schaltuhr (G; die Buchstabenbezeichnungen beziehen sich auf Fig. 1) steuerbares Magnetventil (E), vorzugsweise ein 2-Wege-Magnetventil, die Schaltuhr (G), ein mit dem Magnetventil (E) über ein schlauch- oder rohrartiges Verbindungsstück in Verbindung stehendes Durchflusskontrollventil (H), eine Saugvorrichtung, vorzugsweise eine Vakuumpumpe (F), die an dem Ventil (E) angeschlossen ist, sowie ein Verbindungsstück (D), das vom Durchflusskontrollventil (H) über das Filter (B) zum Sammelrohr (A) bzw. zum Impaktor (Fig. 2) führt. Gemäß einer Ausführungsform der Erfindung enthält die Steuereinheit darüber hinaus ein Mittel zur Messung des Luftvolumenstromes, wobei dieses Mittel zur Messung des Luftvolumenstromes kalibrierbar ist und, vorzugsweise, ein kalibrierbarer mechanischer oder elektrischer Flügelradströmungssensor ist.

Weiter haben die Erfinder gefunden, dass ein besonders gleichmäßiger Volumenstrom erreicht werden kann, wenn an der Einlaufstrecke des Strömungssensors (bzw. dem an diesem angeformten Ansaugrohr) ein Dämpfungsfilter angebracht wird.

Es wurde gefunden, dass durch die Verwendung des kalibrierbaren Sensors, ggf. in Kombination mit einem entsprechend in der Einlaufstrecke platzierten Dämpfungsfilter, Flussschwankungen in der Anzeige von ca. 21 auf 0,1 bis maximal 0,21 herabgesetzt werden können.

In einer weiteren Ausführungsform der Erfindung enthält die Steuereinheit zwei Vakuummessgeräte zur Messung des Unterdrucks auf jeweils einer Seite des Durchflusskontrollventils (Anschlussstellen P 2 und P 3 in Fig. 1 und Fig. 2). Die Luftleitung zu den Vakuummessgeräten erfolgt dabei über jeweils eine Kapillare, die von einem stabilen Außenrohr ummantelt ist. Dieses Rohr dient zum einen als Halterung für das Vakuummessgerät und zum zweiten als Schutz für die Kapillare.

Vorzugsweise enthält die Steuereinheit ein Steuergerät, über das die Steuerung der Ventilöffnung mittels der Schaltuhr erfolgt. Weiter vorzugsweise ist in das Gehäuse des Steuergerätes die Anzeige des Messgerätes für den Flügelradströmungssensor integriert.

Weiter vorzugsweise sind zumindest das Magnetventil, das Durchflusskontrollventil und der Durchflusssensor auf einer gemeinsamen Grundplatte montiert.

Die Ventilsteuerung über das Steuergerät ermöglicht die Einstellung der Ventilöffnung. Variiert werden kann bei einem Ausbringungsvorgang die Dauer der Ventilöffnung. Die eingebaute Flussmessstelle (Flügelradströmungssensor) dient der Flussüberwachung. Die konstruktive Ausgestaltung der erfindungsgemäßen Steuereinheit gewährleistet, dass bei jedem Versuch reproduzierbar die gleiche Luftmenge angesaugt wird und dass keine größeren Druckschwankungen auftreten.

Des weiteren stellt die Erfindung ein Sammelrohr zur Verwendung an einer Steuereinheit gemäß einem der Ansprüche 1 - 5 in Form eines Hohlzylinders bereit, dadurch gekennzeichnet, dass die innere Oberfläche des Sammelrohrs eine Rauhtiefe von maximal 6,3 μm aufweist. Die Rauhtiefe wird erreicht durch den Einsatz spezieller Drehwerkzeuge (Schneidplatten). Der Material- und Werkzeughersteller gibt gewisse Schnittwerte als Richtwerte vor, wodurch die geforderte Rauhtiefe zu erreichen ist.

Außerdem besitzt das erfindungsgemäße Sammelrohr an der einen Seite zwei Einkerbungen zur Aufnahme je eines O-Rings und ist an der anderen, saugseitig gelegenen Seite konisch geformt. Die konische Ausprägung sorgt für einen zweiseitigen Druck auf den Dichtring beim Aufschrauben des Unterteils. Dadurch wird das Saugen von Nebenluft über das Gewinde so gut wie ausgeschlossen.

Ebenfalls Gegenstand der Erfindung ist eine Vorrichtung zur Flussregulierung nach EP/USP, die eine Steuereinheit wie zuvor erläutert, eine Vakuumpumpe und entweder ein Mess- oder Dosis-Sammelrohr oder einen Kaskaden-Impaktor enthält.

Schließlich stellt die Erfindung ein Verfahren zur Bestimmung der Gleichförmigkeit einer aus einem Pulverinhalator ausgebrachten Medikamentendosis bereit, bei dem unter Verwendung der erfindungsgemäßen Vorrichtung in einem ersten Schritt unter Verwendung eines Mess-Sammelrohres die Saugzeit ermittelt wird, mit der das geforderte Saugvolumen erreicht wird, und in einem zweiten Schritt unter Verwendung eines Dosis-Sammelrohres in der ermittelten Saugzeit Pulver aus einem am Dosis-Sammelrohr angebrachten Pulverinhalator in das Dosis-Sammelrohr ausgebracht wird.

Die Erfindung wird nachstehend anhand der Figuren näher erläutert. Dabei zeigt

**Fig. 1** eine Schemazeichnung einer Vorrichtung zur Bestimmung der abgegebenen Wirkstoffmenge gemäß der Europäischen Pharmakopoe, 3. Auflage, Nachtrag 2000;

**Fig. 2** eine Schemazeichnung einer Vorrichtung zur Bestimmung der aerodynamischen Partikelgröße gemäß der Europäischen Pharmakopoe, 3. Auflage, Nachtrag 2000;

**Fig. 3** eine Ansicht der erfindungsgemäßen Vorrichtung aus Steuereinheit zur Flussregulierung mit angeschlossener Saugvorrichtung und Mess-/Dosis-Sammelrohren mit zugehörigen Teilen sowie drei Schnitt- und Ansichtsbilder einzelner Bestandteile der Vorrichtung;

**Fig. 4** eine Ansicht der erfindungsgemäßen Vorrichtung aus Steuereinheit zur Flussregulierung mit angeschlossener Saugvorrichtung und Kaskadenimpaktor mit zugehörigen Teilen;

**Fig. 5** das an P2 oder P3 angebrachte und zum Vakuummessgerät führende Halterohr mit innen verlegter Kapillare im Schnitt;

**Fig. 6** ein Dosis-Sammelrohr (A) und ein Mess-Sammelrohr (B) jeweils im Längs- und Querschnitt;

**Fig. 7** einen Adapter zum Anschluss des Inhalators an das Dosis-Sammelrohr im Längs- und Querschnitt;

**Fig. 8** einen Adapter zum Anschluss unterschiedlich dimensionierter Sammelrohre;

**Fig. 9** einen Saugfinger; und

**Fig. 10** einen Dichtring.

Die in den Abbildungen z.T. angegebenen Abmessungen und Toleranzen sind als Beispiele besonders bevorzugter Ausführungsformen anzusehen und dürfen nicht als den Schutzumfang begrenzend ausgelegt werden.

**Fig. 1** und **Fig. 2** sind Abbildungen aus der Europäischen Pharmakopoe, Nachtrag 2000, und zeigen schematisch den grundlegenden Aufbau von Vorrichtungen zum Ermitteln der Gleichförmigkeit der abgegebenen Dosis von Pulverinhalatoren bzw. zum Ermitteln der aerodynamischen Partikelgröße.

Über eine Vakuumpumpe wird ein Unterdruck erzeugt. Über ein 2-Wege-Magnetventil und ein Durchflusskontrollventil wird der Volumenstrom im Sammelrohr bzw. im Impaktor gesteuert. Vakuumpumpe, Ventile und Sammelrohr bzw. Impaktor sind durch Verbindungsschläuche bzw. Verbindungsstücke miteinander verbunden. Das 2-Wege-Ventil wird durch eine Schaltuhr gesteuert. "P1", "P2" und "P3" bezeichnen die Stellen, an denen Druckmessungen erfolgen können.

Im Einzelnen sind in Fig. 1 folgende Komponenten abgebildet: Die Pumpe F ist mit dem Magnetventil durch einen kurzen und/oder weiten Vakuumschlauch (innerer Durchmesser Di $\geq$ 10 mm) und Verbindungsstücke verbunden. Das 2-Wege-Ventil E öffnet mit geringem Luftwiderstand, muss einen Di von $\geq$ 8 mm und eine maximale Ansprechzeit von 100 ms besitzen. Das Magnetventil E kann über eine Schaltuhr G über die erforderliche Dauer geöffnet werden und ist mit einem Durchfluss-Kontrollventil H über ein Verbindungsstück C (Di $\geq$ 8 mm; z.B. eine kurze Metallverbindung mit einer Abzweigung mit kleinem Durchmesser zum Messpunkt P3) verbunden. Das Durchfluss-Kontrollventil H ist ein Regulierventil mit einem Cv-Wert $\geq$ 1. Hieran schließt sich eine kurze Metallverbindung mit einer Abzweigung mit kleinem Durchmesser zum Messpunkt P2 an. Mit einem Vakuumschlauch D mit Di = 8 +/- 0,5 mm und Länge L = 50 +/- 10 cm wird das Sammelrohr bzw. der Impaktor an die Steuereinheit angeschlossen. Zur Einstellung des Unterdruckes ist ein Vakuumschlauch an P 1 am Sammelrohr A mit einem Manometer zu verbinden. Der angelegte Volumenstrom lässt sich durch eine Verbindung (Adapter/Siliconschlauch) am Eingang des Sammelrohrs A zum Flügelradströmungssensor messen. Gemäß der gezeigten Abbildung soll das Sammelrohr einen inneren Durchmesser von 34,85 mm und eine Länge von 12 cm besitzen. An der saugseitigen Rückwand ist das Sammelrohr A mit einem Filter B, z.B. einem Glasfaserfilter, versehen. An den saugseitig bzw. Sammelrohr-seitig angeordneten Druckmessstellen P3 und P2 wird der Absolutdruck gemessen, an P 1 der Differenzdruck gegenüber dem atmosphärischen Druck.

In Fig. 2 ist die Vakuumpumpe mit D, das Magnetventil mit C, die Schaltuhr mit E, das Verbindungsstück mit A, das Durchflusskontrollventil mit F und der Verbindungsschlauch mit B bezeichnet. Anstelle des Sammelrohrs A in Fig. 1 ist ein Impaktor an den Verbindungsschlauch B angeschlossen.

Die Funktionsweise der in Fig. 1 und 2 gezeigten Vorrichtungen ist in der Einleitung erläutert.

**Fig. 3** zeigt eine erfindungsgemäße Vorrichtung mit einer erfindungsgemäßen Steuereinheit, einer daran angeschlossenen Saugvorrichtung 83 (z.B. einer Vakuumpumpe 84) und einem über die Schlauchverbindung 40 an die Steuereinheit angeschlossenen Mess-Sammelrohr. (Die Schlauchverbindung 39 wird zur Einstellung des Druckabfalls, die Schlauchverbindung 38 zur Bestimmung des angelegten Volumenstroms hergestellt.) Rechts neben der Apparatur sind ein Mess-Sammelrohr 68 und ein Dosis-Sammelrohr 60 mit weiteren Anschlussteilen gezeigt. Die Schnittzeichnungen durch die Ebenen A-A und B-B zeigen einen Sammelrohrhalter bzw. einen Flügelradmesssensor mit Anschlussteilen. Ansicht X zeigt ein Vakuummessgerät mit Anschlussteilen in der Seitenansicht.

An die Steuereinheit wird zur Erzeugung des erforderlichen Soges eine Saugvorrichtung 83, z.B. eine Vakuumpumpe 84 angeschlossen. Um die Steuereinheit mit möglichst geringen Flussschwankungen betreiben zu können, ist ein entsprechendes Saugvolumen erforderlich. Voraussetzung hierfür ist daher eine geeignete Vakuumpumpe mit einem ausreichenden Saugvermögen. Geeignet ist z. B. die Vakuumpumpe ME 16 oder die chemoresistente ME16 C der Fa. Vacuubrand GmbH, Wertheim. Die Wahl ist abhängig von der jeweiligen Belastung durch eingesaugte Gase oder Lösungsmitteldämpfe. Hierbei handelt es sich um eine einstufige trockenverdichtende Membran-Vakuumpumpe. Durch die Verschaltung der einzelnen Pumpenköpfe wird ein Saugvermögen von bis zu 10,1 m$^3$/h erreicht. Bei der genannten Pumpe öffnen und schließen die Flatterventile durch die Gasströmung selbsttätig. Wird am Auslassstutzen zum Absaugen schädlichen Gase oder Lösungsmitteldämpfen ein Schnüffelvakuum angeschlossen, so ist darauf zu achten, dass dies nicht angelegt ist, wenn die Pumpe außer Betrieb ist, da hierbei die Flatterventile Schaden nehmen könnten. Die Pumpe erreicht die volle Saugleistung und den Enddruck erst bei erreichter Betriebstemperatur. Dies ist in der Regel nach ca. 15 Minuten der Fall. Eine Mindestvorwärmzeit von 15 Minuten ist daher zu berücksichtigen. Die Betriebsanleitung des Geräteherstellers ist zu beachten.

Alternativ können auch andere Saugvorrichtungen eingesetzt werden, solange der erforderliche "kritische Durchfluss" (s. unten) erreicht wird.

Die Vakuumpumpe ist mit einem Vakuumschlauch 82, z. B. einem formstabilen Kautschuk-Vakuumschlauch (DN 20, $D_a$ = 45 mm, $d_i$ =19 mm) mit der Steuereinheit verbunden.

Die Steuereinheit selbst besteht aus mehreren Einzelteilen:

Ein Magnet-Ventil 27 gesteuert über eine Zeitschaltuhr (Timer) dient dazu, über eine vorgegebene Zeit bei konstantem Volumenfluss ein definiertes Saugvolumen durch den Inhalator und das Dosis-Sammelrohr (bzw. den Kaskadenimpaktor) zu schalten. Es eignet sich u.a. ein Magnetventil der Fa. Bürkert, Filderstadt (Best.Nr. 062 347 E). Das Gehäuseteil ist hierbei aus Edelstahl (1.4581), die Schaltzeiten beim Öffnen liegen zwischen 10 - 20 ms, beim Schließen zwischen 20 - 30 ms, die Nennweite beträgt 10 mm, der $C_v$ - Wert 1,5 m$^3$/h (Wasser). Die Spannung beträgt 24 V und die Frequenz 50 Hz.

Geeignet sind jedoch auch alle anderen Ventile, die die Anforderungen und Vorgaben der USP/EP erfüllen, nämlich einen inneren Durchmesser von gleich oder größer 8 mm und eine maximale Ansprechzeit von kleiner 100 ms besitzen.

Außerdem sind in Fig. 3 im Zusammenhang mit dem Ventil 27 ein Haltewinkel 25 und eine Schlauchtülle 16 für das Ventil 27 sowie Befestigungsmittel 41 und 96 gezeigt.

Ein Durchflusskontrollventil 33 mit Regulierhahn dient zur manuellen Einstellung des erforderlichen Druckabfalls. Mit diesem Durchflusskontrollventil 33 wird - bei geöffnetem Magnetventil 27 - ein definierter Druckabfall, insbesondere ein Druckabfall von 4,0 kPa (Vorgabe aus EP/USP) eingestellt. Dies entspricht umgerechnet 40,8 cm Wassersäule, abgelesen am Lineal 26 des Manometer 22, 29. Der angelegte Unterdruck wird, wie später noch im Detail erläutert wird, mit Hilfe eines Mess-Sammelrohrs 68 und daran angeschlossenem Differenzdruck-Messinstrument (Manometer) 22, 29 eingestellt.

Als Durchflusskontrollventil 33 wird beispielsweise ein Feinreguliernadelventil der Fa. Hoke mbH, 60314 Frankfurt, eingesetzt, das einen $C_v$ -Faktor von 1,2 besitzt. Es eignen sich jedoch auch andere Ventile, solange der von der EP/USP geforderte $C_v$ -Wert von größer 1 gewährleistet ist.

Außerdem gezeigt ist ein Halter 24 für das Durchflusskontrollventil 33.

Auf beiden Seiten des Durchflusskontrollventils 33 befindet sich ein Anschluss 18 (hier mit Doppelnippel) bzw. 17 (hier mit Schlauchtülle) für je ein Druck-/Vakuummessgerät 36. Mit diesen, neben dem Durchflusskontrollventil angebrachten Druck/Vakuummessgeräten 36 wird der absolute Druck auf beiden Seiten des Durchflusskontrollventils 33 gemessen. Ist das Druckverhältnis zwischen den Druckmesspunkten P3 und P2 (vgl. Fig. 1 und Fig. 2) gleich oder kleiner 0,5, so ist die Forderung nach dem "kritischen Durchfluss" laut USP/EP erfüllt. Bei angelegtem Fluss muss der oben genannte Wert mindestens erreicht werden. Wird dieser nicht erreicht, ist eine kräftigere Saugeinheit anzuschließen.

Als Druck-/Vakuummessgeräte 36 können beispielsweise die vollelektronischen Vakuummessgeräte der Fa. Vacuubrand, Wertheim, eingesetzt werden. Die Geräte haben laut Hersteller einen Messbereich von 1 bis 1080 mbar (Messgenauigkeit nach erfolgtem Abgleich und gleichbleibender Temperatur von $\leq \pm$ 1 mbar).

Außerdem enthält die Steuereinheit ein Steuergerät 89. Dieses enthält eine Anschlussbuchse für das 2-Wege-Magnetventil 27, ggf. (mit Kontrollleuchte), eine Zeitschaltuhr (vorzugsweise eine Digitalzeitschaltuhr), nachfolgend auch "Timer" oder "Schaltuhr" genannt, zur Ansteuerung des 2-Wege-Magnetventils 27 sowie einen Auslöser zum Starten der Zeitschaltuhr (z. B. eine Start-Taste). Die Schaltuhr ist vorzugsweise für Messungen mit einem Einstellbereich von 0,001 s bis z. B. 9999 h (Einstellgenauigkeit bei der Messung von 2 Stellen hinter dem Komma, Toleranzgrenze ± 0,3s bei Kalibrierbereich von 5-7s) geeignet. Optional enthält das Steuergerät 89 außerdem eine Netzkontrollleuchte, eine Anschlussbuchse für den Flügelradströmungssensor 37, eine Anzeige für den Durchfluss am Flügelradströmungssensor 37 sowie einen (Kipp-)Schalter für wahlweise Dauer- oder Zeitbetrieb des Magnetventils 27.

Das Steuergerät 89 wird vorzugsweise über ein Halteblech 88 und die Grundplatte 1 fest mit den restlichen Komponenten der Steuereinheit verbunden. Dies ist vorteilhaft, da Steuereinheit und Flügelradströmungssenor als zusammengehörige Einheit kalibriert werden müssen. Durch die feste Verbindung der Komponenten miteinander wird die Zuordnung der einzelnen Teile zueinander gewährleistet. Die Bezugszeichen 41, 50, 56, 97 und 102 bezeichnen Teile von Befestigungsmitteln (z.B. 41 und 56: Linse Flanschkopfschraube mit Innensechskant; 50: Unterlegscheibe; 97: Hutmutter; 102: Sechskantmutter).

Das Manometer bestehend aus 22, 29, 7, 26 und diversen anderen Teilen ist als Differenzdruck-Messinstrument ausgebildet und dient der Anzeige und Einstellung des vorgegebenen Druckabfalls (4,0 kPa). Die Einstellung des Druckabfalls erfolgt immer in Verbindung mit dem jeweiligen Device (z.B. dem Inhalator "HandiHaler®" 80) oder einem geeigneten Ersatzwiderstand 66, welcher den Strömungswiderstand, z.B. den Inhalator "HandiHaler®" 80 mit eingelegter Kapsel, simuliert. Das Manometer besteht z.B., wie in Fig. 3 gezeigt, aus einem Metallrohr 22, aus einem Glas-Steigrohr 29 mit verschiebbarem Lineal 26 (z.B. 50 cm) zur Messung der Wassersäule. Das Steigrohr 29 taucht in ein mit Wasser gefülltes Gefäß 31, dessen Durchmesser mindestens das 20-fache des Innendurchmesser des Steigrohres 29 haben sollte (z.B. eine Kristallisierschale). Zur besseren Benetzung des Steigrohres 29 werden einige Tropfen Spülmittel dem Wasser zugesetzt. Am Steigrohr 29 ist ein Metallrohr mit Olive 13 fest verschraubt. Das Steigrohr 29 (Glasrohr) wird von Seiten des Herstellers zusammen mit einem Dichtring geliefert, der sich beim Anziehen der Verschraubung zusammenziehen und so das Glasrohr mit dem Metallrohr dicht verbinden soll. Es hat sich jedoch gezeigt, dass mit einer solchen Verbindung keine hinreichende Abdichtung erzielt werden kann und außerdem beim Nachziehen der ursprünglichen Dichtung das Glasrohr leicht im Gewinde zerspringt. Es erwies sich als vorteilhaft, stattdessen einen kleinen Flansch an das Metallrohr zu löten und hierauf einen O-Ring derart aufzusetzen, dass beim Verschrauben das Glasrohr auf der oberen Kante gegen den Flansch abgedichtet wird.

Bei der Messung des Druckabfalls im Mess-Sammelrohr 68 wird an der Metallolive 13 über den Vakuumschlauch 39 (z.B. Di = 8 mm, L = 35 cm) das Mess-Sammelrohr 68 mit dem Anschlussstutzen 69 (Position "P1" in Fig. 1) verbunden. Bei der Messung ist darauf zu achten, dass das Gefäß 31 (in der Höhe mit Hilfe Vorrichtung 32 justierbar, z.B. einem Laborständer) mit einer ausreichenden Menge Wasser gefüllt ist. Das Steigrohr 29 muss in das Wasser eintauchen und der Nullpunkt des (ggf. auf die Nullmarke abgeschnittenen) Lineals 26 wird durch Verschieben auf die Höhe des unteren Meniskus in dem Steigrohr oder Vorrichtung 32 eingestellt. Das Lineal 26 lässt sich durch Lösen und wieder Anziehen der Befestigungsmittel justieren.

Weiter sind in Fig. 3 mehrere Halter 35 für das Manometer (bestehend aus 22, 29, 7, 26 und diversen anderen Teilen), versehen mit Befestigungsmitteln 43 und 52 (z.B. Senkschraube und Sechskantmutter), sowie Linealhalterungen 4 mit einer Scheibe 49 am Linealhalter und Befestigungsmitteln 44 und 53 (z.B. Senkschraube und selbstsichernde Sechskantmutter), ein O-Ring 85, Halter 6 mit Befestigungsmitteln 44, Rändelhohlmuttern 34 für den Linealhalter, Abstandhalter 5, ein Montageblech 7 sowie weitere Befestigungsmittel 41 gezeigt.

Wie erwähnt kommt das Manometer bestehend aus 22, 29, 7, 26 usw. in Verbindung mit dem Mess-Sammelrohr 68 zum Einsatz.

Der Flügelradströmungssensor 37 dient zur Messung und Kontrolle des eingestellten/einzustellenden Volumenstroms. Der Sensor 37 ist über ein Rohranschlussstück 15 und eine Dichtung 93 übergangslos mit einem Saugrohr 14 verbunden, das endständig eine Gewindehülse 92 und einen Schlauchanschluss 30 (z. B. G3/8 1/3 Verschraubung) aufweist. Um exakte Messungen des Volumenstroms durchführen zu können, muss der Flügelradströmungssensor kalibrier- und justierbar sein.

Der Messbereich (Luft/Gas) des Flügelradströmungssensor liegt zwischen 0,4 und 20 m/s, die Temperaturbeständigkeit bei von -20°C bis + 100°C. Überprüft wird z.B. bei 5 verschiedenen Werten (28,3, 35,0, 39,0, 45,0 und 50,0 l/min). Als Toleranz werden $\pm$ 1 l/min und als Warngrenze 0,8 l/min angegeben. Die bisherigen Prüfberichte aus der Praxis haben gezeigt, dass die Fehler/Abweichungen bei $\pm$ 0,1 bis max. 0,4 l/min liegen.

Im Stand der Technik verwendete Flowmeter zeigen Abweichungen von mindestens 2 %, d.h. 2,4 l/min (Prospekt der Fa. Copley). Mit dem erfindungsgemäß in Verbindung mit der Steuereinheit verwendeten Flügelradströmungssenor muss demgegenüber maximal mit Abweichungen von 0,7 l/min, in der Regel nur mit Abweichungen von 0,1 - 0,4 l/min gerechnet werden. Hierdurch wird die Messgenauigkeit erheblich verbessert.

Der Flügelradströmungssensor 37 und das Saugrohr 14 sind über einen Halter 8, einen Gewindebolzen 9, einen Gewindestift für Saugrohrhalterungen 48 und eine Schraubrohrschelle 28 mit einem Stativ 2, verankert auf der Grundplatte 1 über eine Fußplatte 3, verbunden. Bezugszeichen 47 bezeichnet ein Befestigungsmittel, z.B. eine Senkschraube. Das Saugrohr hat bestimmte Abmaße der Ein- und Auslaufstrecke, um entstehende Turbulenzen des Luftstroms zu glätten. Insbesondere ist der Übergang vom Saugrohr zum Flügelradströmungssenor so gearbeitet, das keine Querschnittsverjüngung entsteht und nur eine kleine Naht im Verbindungsstück vorliegt. Hierdurch kann verhindert werden, dass vor dem Flügelrad Strömungsturbulenzen entstehen.

Die konstruktive Ausgestaltung des Flügelradströmungssenors und der daran anschließenden Rohre und Verbindungen gewährleistet, das der Fluss immer an der gleichen definierten Stelle gemessen wird. Durch definierte Ein- und Auslaufstrecken werden die Messgenauigkeit und die Zuverlässigkeit erhöht.

Optional kann am Saugrohr ein Schutzgitter angebracht werden, das verhindern soll, dass kleinere, leichte Gegenstände in das Saugrohr hineingezogen werden. Es ist dann jedoch zu prüfen, in welchem Ausmaß die Luftströmung durch ein solches Gitter beeinflusst wird. Erfindungsgemäß hat sich jedoch stattdessen das Anbringen eines Dämpfungsfilters, befestigt in einer Delrin-Dichtscheibe (104, 105) und angeschraubt mit einer Überwurfmutter als besonders vorteilhaft erwiesen. Hierdurch kann erreicht werden, dass die Flussanzeige, die ohne Einsatz des Dämpfungsfilters z.B. 2 l/min schwanken kann, mit dem Dämpfungsfilter nur noch einer Schwankung von 0,1 bis maximal 0,2 l/min unterliegt.

Der Flügelradströmungssensor 37 wird in Verbindung mit einem Mess-Sammelrohr 68 eingesetzt und über einen Schlauch 38, z.B. aus Silicon mit Di = 12 x 2,5 mm und L = 50 cm, mit diesem über einen Adapter 64 verbunden.

In der in Fig. 3 gezeigten Ausführungsform der erfindungsgemäßen Vorrichtung wird die Steuereinheit entweder - zur Einstellung des Unterdrucks - mit einem Mess-Sammelrohr 68 oder - zur Durchführung der eigentlichen Messung, also der Bestimmung des Prüfpunktes "abgegebene Dosis/Wirkstoffmenge" - mit einem Dosis-Sammelrohr 68 verbunden. In beiden Fällen erfolgt der Anschluss des Sammelrohrs an das Durchflusskontrollventil 33 und den Vakuummessgerätanschluss 17 über einen Vakuumschlauch 40, der z.B. aus Gummi gefertigt ist.

Mess-Sammelrohr:

Das Mess-Sammelrohr 68 dient zur Einstellung des Unterdruckes und zur Ermittlung des sich daraus ergebenden Volumenstroms. Hieraus wird die Zeit für den erforderlichen Volumenfluss, z.B. 41 berechnet. Dies erfolgt unter Verwendung des Differenzdruck-Messinstruments/Manometers 22, 29 usw. , eines geeigneten Ersatzwiderstands 66 sowie des Flügelradströmungssensors 37. Das Mess-Sammelrohr 68 hat die gleichen Abmessungen wie das Dosis-Sammelrohr 60, unterscheidet sich von diesem jedoch durch das Vorhandensein einer Anschlussolive 69 am Anschlusspunkt P1 (Fig. 1). Über diese wird zur Einstellung des Druckabfalls das Mess-Sammelrohr über einen Vakuumschlauch 39, der z.B. aus Gummi gefertigt ist und die Abmessungen Di = 8mm, L = 35 cm besitzt, mit dem Manometer 22, 29 usw., an der Schlauchtülle 13 verbunden.

Die Anschlussolive wird vorzugsweise so in das Mess-Sammelrohr eingepresst, dass durchgängig bis zur Sammelrohrinnenseite der in der USP/EP angegebene Durchmesser von 2,2 mm gegeben ist. Bei größeren Öffnungen (z.B. 8 - 10 mm) kann der Luftstrom im Sammelrohr durch Turbulenzen gestört werden, was zu deutlichen Schwankungen des am Manometer abzulesenden Drucks führt.

Im Unterteil des Sammelrohres (gleichermaßen bei Mess- und Dosis-Sammelrohr) wird eine Lochplatte 57 zur Stützung des Glasfaserfilters 95 eingelegt. Hierbei ist die offenporige Seite der Lochplatte 57 nach oben und die "Lochseite" (glatte Seite der Lochplatte, saugseitig) nach unten anzubringen. Eine falsch eingelegte Lochplatte 57 macht sich durch eine deutliche Erhöhung des Strömungswiderstandes bemerkbar. Die Lochplatte sorgt konstruktionsbedingt für einen gleichmäßigen Sog auf dem ganzen Glasfaserfilter. Ein Reißen im Zentrum des Filters durch Sogeffekte, wie bei Röhren andere Anbieter beobachtet, ist ausgeschlossen.

Auf der Lochplatte 57 werden das Glasfaserfilter 95 und ein O-Ring 70 positioniert. Der O-Ring 70 dient zum Halten des Filters 95 und Abdichten des verschraubten Unterteils 61 mit dem Sammelrohr 60 bzw. 68. Bedingt durch den unter dem Gewinde des Sammelrohrs 60 bzw. 68 angebrachten Konus wird das Filter durch den O-Ring auf das Lochsieb gedrückt und zugleich seitlich im Unterteil das Gewinde gedichtet. Dadurch ist bei richtigem Zusammenbau ein Ziehen von "Nebenluft" unmöglich. Beim Einsatz des Sammelrohrs wird saugseitig das Unterteil 61 (mit O-Ringen 86) über den Verbindungsadapter 58 (innerer Durchmesser z.B. 8 mm an der engsten Stelle) über den Vakuumschlauch 40 (Länge z.B. 45 cm, Di = 8,0 mm ± 0,5 mm) an die Steuereinheit angeschlossen.

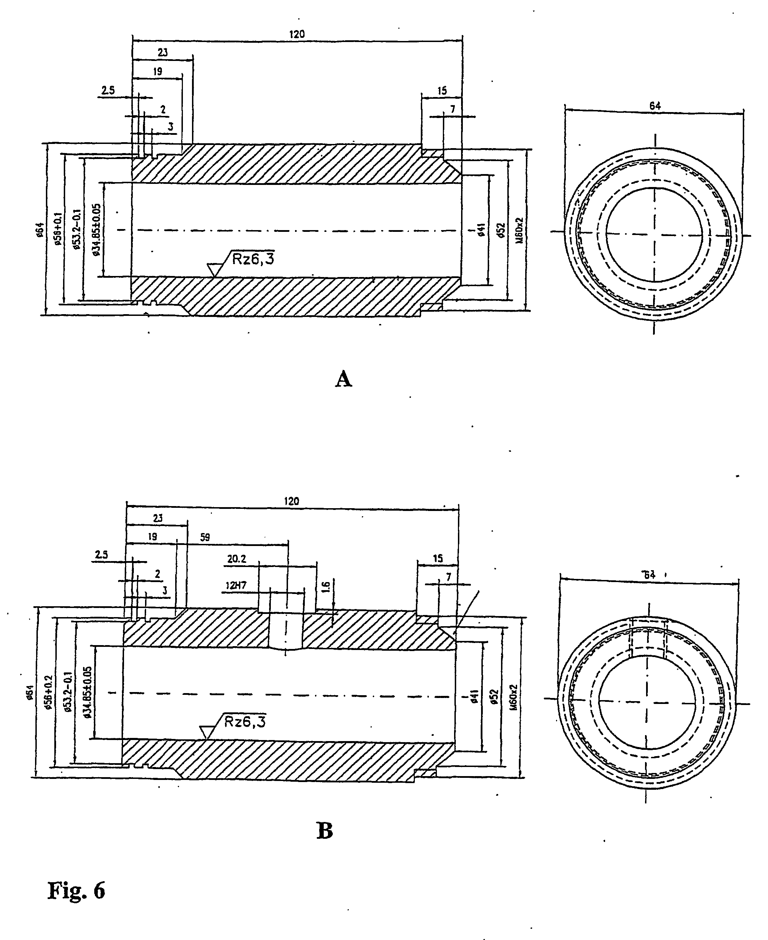

Wie in Fig. 6A und Fig. 6B gezeigt ist, befinden sich am Probeneingangsende des Sammelrohres zwei O-Ringe 71, durch die Dichtigkeit und fester. Sitz gewährleistet werden. Hieran schließt sich ggf. ein Sammelrohradapter 62 bzw. ein Ersatzwiderstand 67 an, der entweder für das entsprechende Inhalator-Device (z.B. den "HandiHaler®" 80) ausgelegt ist (Sammelrohradapter 62) oder mit einem integrierten geeigneten Ersatzwiderstand 66 versehen ist (Sammelrohradapter 67), welcher den Strömungswiderstand, z.B. des Inhalators "HandiHaler®" 80 mit eingelegter Kapsel, simuliert. Eine Einstellung des Unterdruckes mit Inhalator und eingelegter Kapsel wie nach EP/USP vorschlagen ist im Fall des HandiHaler bedingt durch die Vibration der Kapsel nicht möglich. Die Wassersäule würde zu sehr schwanken und nicht exakt auf 40,8 cm einstellbar sein. Das Device bzw. der Ersatzwiderstand beeinflussen entscheidend den erforderlichen einzustellenden Unterdruck und die jeweils sich hieraus ergebende Flusseinstellung für die spätere Bestimmung. Die Ausführung des erforderlichen Inhalator-Adapters 62 für das Sammelrohr und des Adapters 67 mit integriertem Ersatzwiderstand 66 ist davon abhängig, welcher Inhalatortyp eingesetzt wird (näheres siehe weiter unten). Die Verwendung zweier hintereinanderliegender O-Ringe erhöht die Dichtigkeit und den festen Sitz. Bei Verwendung nur eines O-Rings ist die Verbindung zum Adapter weniger stabil und wird leichter undicht.

Außerdem in Fig. 6A und 6B gezeigt ist das konisch verlaufende saugseitige Ende des Sammelrohrs, das über einen Silikonring dicht mit dem Unterteil 61 verbunden wird. Der Konus drückt dabei den eingelegten Silikonring an die Wandung und das Filter, wodurch ein Ansaugen von "Nebenluft" durch das Gewinde bzw. die Verschraubung verhindert wird.

Außerdem gezeigt sind die Verschlusskappe 59 für das Sammelrohr sowie die Schutzkappe 65 für den Ersatzwiderstand 66.

Dosis-Sammelrohr:

Für die Bestimmung "Gleichförmigkeit der abgegebenen Dosis" bei Pulver zur Inhalation ist das Dosis-Sammelrohr 60 ein wesentliches Hilfsmittel. Es dient hierbei zur Aufnahme der aus dem Inhalator-Device bzw. der darin enthaltenen Pulverkapsel ausgebrachten Pulvermenge. Das Dosis-Sammelrohr 60 wird beispielsweise aus Kunststoff (Delrin) gefertigt und besitzt insbesondere die von der EP/USP exakt vorgeschriebene Innenlänge von 120 mm und einen inneren Durchmesser von 34,85 mm. Typischerweise werden mehrere Dosis-Sammelrohre vorgehalten werden. Diese sind dann zwecks Identifizierbarkeit und Passgenauigkeit individuell zu kennzeichnen. Weitere Werkstoffe zur Herstellung der Sammelrohre sind möglich, wie z. B. Teflon. Nachteil wäre der höhere Preis des Werkstoffes, die schlechtere "Spanfähigkeit" und schwierigere Oberflächenbearbeitung im Sammelrohr. Bei den hohen Anforderungen an die Passgenauigkeit und Dichtigkeit sind Teflonverbindungen "schwergängiger". Sollte Teflon erforderlich sein, ist eine Kombination von Delrin/Teflon-Verbindungen vorteilhaft, da Teflon durch das "weiche" Delrin leichtgängiger wird. Alternativ können auch andere Werkstoffe verwendet werden. Diese sind jedoch auf Kompatibilität mit den zu bestimmenden Wirkstoffen und eingesetzten Lösungsmitteln zu überprüfen.

Die Porosität der eingesetzten Glasfaserfilter 95 hat einen entscheidenden Einfluss auf die Wirkstoffrückhaltung und beeinflusst den Strömungswiderstand des Dosis-Sammelrohrs 68 und somit auch die Betriebsparameter der Steuereinheit. Insbesondere sind die Auswirkungen auf die erforderliche Saugleistung der Saugeinheit zu beachten.

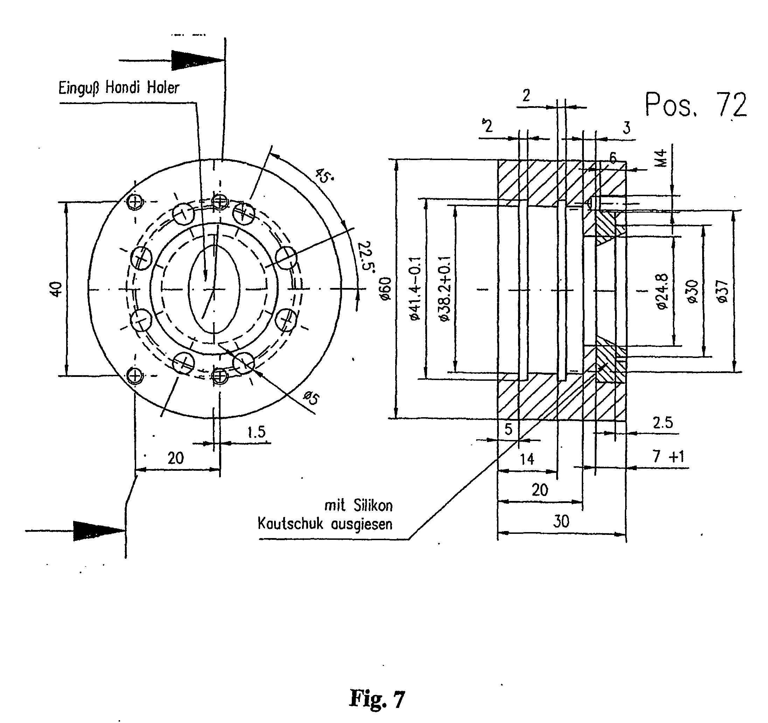

Das saugseitige Verschlussteil des Dosis-Sammelrohres 60 hat den gleichen Aufbau wie das des Mess-Sammelrohrs. Die Abmessungen des Dosis-Sammelrohres 60 entsprechen denen des Mess-Sammelrohres 68. Das Dosis-Sammelrohr 60 besitzt jedoch keine Anschlussolive für das Manometer (P1), wodurch verhindert wird, dass sich Teile des eingebrachten Pulvers an einer derartigen Anschlussstelle verfangen und von der quantitativen Bestimmung der eingebrachten Pulvermenge nicht mehr erfasst werden. Der Eingang des Dosis-Sammelrohres 60 (mit O-Ring 71) wird beim Vorgang der Ausbringung mit einem Inhalator-Adapter 62 versehen, der zur Aufnahme des jeweiligen Inhalator-Typs dient und auf diesen abgestimmt ist. Der Adapter 62 soll einen spannungsfreien, dichten Sitz gewährleisten. Im Falle des Inhalators "HandiHaler®" ist beispielsweise darauf zu achten, dass die Siliconmasse i. d. R nicht dicker als $7 \pm 1$ mm sein soll, damit Passgenauigkeit und Dichtigkeit gewährleistet sind. Wie in Fig. 7 gezeigt, ist der Adapter 62 mit einem Einguss versehen, der sicherstellt, dass die Vorderseite des Inhalator-Mundstückes in gleicher Ebene mit dem Dosis-Sammelrohr abschließt und das Mundstück vollständig dicht anschließt. Der Siliconeinguss selbst wird durch warzenförmige Ausformungen im Adapterprofil gehalten und somit im Deckel fixiert. Dies wird durch Bohrlöcher von 5 mm im Adapter erreicht. Die Siliconmasse setzt sich in die Ausbohrung und sichert nach dem Aushärten der Siliconmasse den Einguss gegen das Verdrehen.

Außerdem gezeigt ist in Fig. 3 ein Winkel 94 für den Inhalator-Adapter 62 (mit Befestigungsmittel 98), ein Blindstopfen 63, der zum Verschließen der Sammehohere bei der Aufarbeitung der Probe dient, sowie der Inhalator "HandiHaler®" 80 mit Stützstift 99 zur stabilen Befestigung des "HandiHaler®" 80 am Inhalator-Adapter 62. Es können auch andere Inhalatoren eingesetzt werden. Es sind dann jeweils an diese anderen Inhalatoren angepasste Sammelrohr-Adapter zu verwenden.

Die Herstellung der Mess- und Dosis-Sammelrohre sowie der Adapter erfolgt vorzugsweise aus Delrin® (auch als Polydelrin oder POM bezeichnet). Delrin® ist ein Polyoxymethylen (POM) und ist eine Alternative zu Teflon®, das in der Pharmakopoe als Material genannt wird. Letztlich ist das Material in Abhängigkeit vom in die Bestimmung einzusetzenden Wirkstoff zu wählen: Wechselwirkungen des Materials mit dem Wirkstoff müssen ausgeschlossen werden. Daher ist für jeden Wirkstoff die Kompatibilität mit dem o. g. Herstellungsmaterial zu überprüfen. Weitere Werkstoffe für Sammelrohre sind denkbar, wobei jedoch stets darauf zu achten ist, dass erstens die in der EP/USP angegebenen Abmessungen und Anforderungen erfüllt sind und zweitens Kompatibilität mit den Wirkstoffen gegeben ist. Werden organische Lösungsmittel eingesetzt, z. B. Methanol -WasserGemische, so muss der Werkstoff auch bezüglich dieser Materialien auf Eignung und Materialverträglichkeit überprüft werden.

Bei der Herstellung eines Sammelrohrs haben die Einzelteile gewisse Fertigungstoleranzen. Deshalb sind alle Teile jedes Sammelrohres zu kennzeichnen. Es dürfen nur zusammengehörige Teile eingesetzt werden. Bei Reparaturen bzw. Neuanfertigungen von Einzelteilen muss das gesamte Sammelrohr einbezogen werden. Der Grund hierfür ist, dass die Passgenauigkeit und Kompatibilität mit anderen Adaptern gewährleistet sein muss.

Es hat sich im Rahmen der Erfindung als vorteilhaft erwiesen, Fertigungsteile mit engeren Toleranzen als nach DIN 7168-m gefordert herzustellen. Durch die fertigungstechnisch enger festgelegten Toleranzen wird eine hohe Passgenauigkeit erreicht, die zu einem konstanteren Luftvolumenfluss und damit zu genaueren Messergebnissen beiträgt.

Der Schnitt A-A in Fig. 3 zeigt einen Sammekohrhalter 10 mit Seitenteilen 11 und Gummibändern 21 zur Befestigung des Sammelrohrs. Die Befestigung der Gummibänder 21 erfolgt dabei z.B. an der einen Seite fest über eine Unterlegscheibe 50, ein Befestigungsmittel 56 (z.B. eine Linsen-Flanschkopfschraube mit Innensechskant) und eine Blechplatte 103. Auf der gegenüberliegenden Seite erfolgt die Verbindung zweckmäßigerweise lösbar, z.B. über Zylinderschrauben 90, Hohlnieten 91 für das Gummiband, Scheiben 51 für das Gummiband und Sechskantmuttern 54. Alternativ besteht die Möglichkeit, an Stelle der Gummibänder 21 ein Klettband mit entsprechenden Abmessungen zu verwenden. Auf der gegenüberliegenden Seite erfolgt dann die Verbindung zweckmäßigerweise mit einem z.B. aufgeklebten Klettbandstück, so dass die Zylinderschrauben 90, die Hohlnieten 91 für das Gummiband, die Scheiben 51 für das Gummiband und die Sechskantmuttern 54 entfallen.

Sammelrohrhalter 10 und Seitenteile 11 sind über ein Befestigungsmittel 45 miteinander verbunden. Die Seitenteile 11 sind über eine Fußplatte 12 und Befestigungsmittel 45 und 46 mit der Grundplatte 1 verbunden.

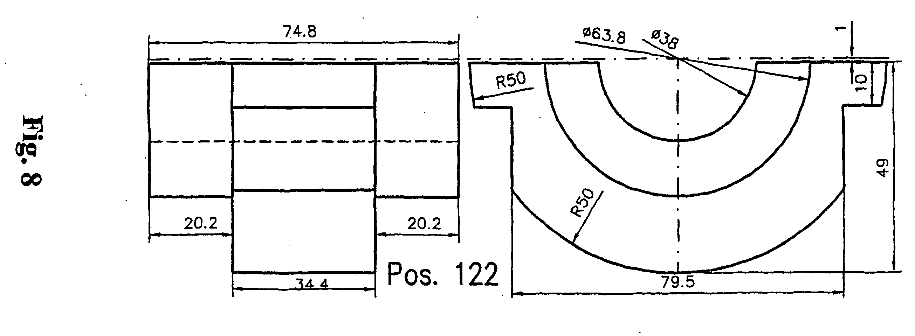

Der Sammelrohrhalter kann mit einem Adapter 122 (**Fig. 8**) versehen werden, der es ermöglicht, auch Sammelrohre mit anderen Abmessungen (z.B. die kleineren Rohre für Aerosole und Lösungen) in den Halter einzuspannen und an der erfindungsgemäßen Steuereinheit zu betreiben.

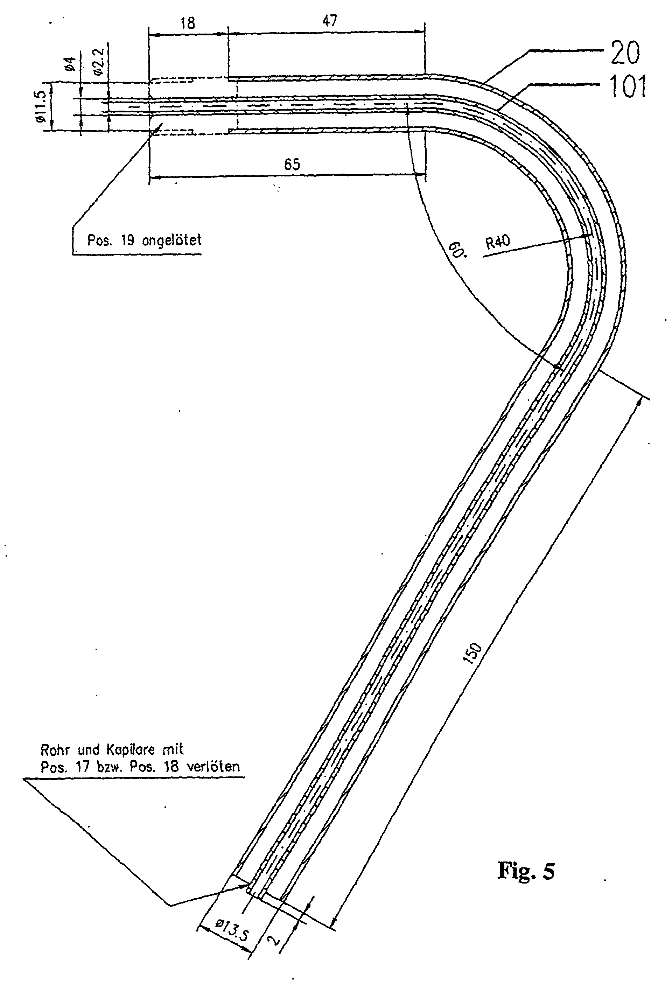

Schließlich zeigt Ansicht X der Fig. 3 ein Vakuummessgerät 36, das über einen Gewindenippel 19 und ein Halterohr 20 mit dem Vakuummessgeräteanschluss 17 verbunden ist. Wie in **Fig. 5** gezeigt ist, befindet sich im Halterohr 20 eine dünne Kapillare 101. Durch Verlötung des Rohres 20 und der Kapillare 101 mit den Positionen 17 bzw. 18 werden Leckagen ausgeschlossen. Außerdem besitzt das geringere Volumen des Kapillareninhalts den Vorteil, dass bei der Messbetriebseinbindung des Vakuummessgerätes geringe bis nicht messbare Volumenverluste durch die Kapillaren verursacht werden. Das geringe Luftvolumen wirkt sich also bei der Messung nicht auf das zu ziehende Saugvolumen aus. Das Außenrohr dient einerseits als Halter für das Messgerät und andererseits zum Schutz der eingelegten Kapillaren. Ebenfalls in **Fig. 3** gezeigt ist das Durchflusskontrollventil 33, ein Gewindestift 42 zur Fixierung des Durchflusskontrollventils, ein Halter 24 für das Ventil und ein Befestigungsmittel 47 zur Verankerung auf der Grundplatte 1.

In der gezeigten Ausführungsform sind das Magnetventil 27, das Durchflusskontrollventil 33, der Sammelrohrhalter 10, 11, 12 direkt, der Flügelradströmungssensor 37 über das Stativ 2 sowie das Steuergerät 89 und das Manometer 22, 29 über Haltebleche auf der Grundplatte 1 verankert. Diese enthält an ihrer Unterseite Schwingmetallpuffet 55. Schließlich ist auf der Grundplatte 1 eine Impaktor-Standschale 23 befestigt, die bei Verwendung der Steuereinheit mit einem Impaktor zum Einsatz kommt (vgl. Erläuterungen zu Fig. 4). Durch die metallische Ausgestaltung und die Verschraubungen aller Bauteile miteinander können keine Bezugserdpotentiale auftreten. Über das Steuergerät und die Anschlußkabel der Steckdose ist die gesamte Steuereinheit in sich geerdet. Überspringende statische Aufladungen werden ebenfalls abgeleitet. Jedes Gerät hat ein EMV Zertifikat. Die Steuereinheit wird zweckmäßigerweise für gängige Betriebsstromarten (220 V und 110 V) gebaut und die elektrischen Module werden auf die jeweilige Betriebsstromart ausgelegt. Die Module auf der Steuereinheit können beschriftet werden, z.B. in Deutsch oder Englisch.

**Fig. 4** zeigt eine erfindungsgemäße Vorrichtung mit einer erfindungsgemäßen Steuereinheit, einer daran angeschlossenen Saugvorrichtung 83 (z.B. einer Vakuumpumpe 84) und einem über die Schlauchverbindung 40 an die Steuereinheit angeschlossenen (Kaskaden-) Impaktor 75. Rechts neben der Apparatur sind ein "HandiHaler®" 80 und weitere Anschlussteile gezeigt. In Fig. 4 erscheinende Bezugzeichen, die bereits in Fig. 3 verwendet wurden, besitzen in Fig. 4 die gleiche Bedeutung wie in Fig. 3 und werden nachstehend nur teilweise erneut erläutert.

Die gezeigte Apparatur dient zur Bestimmung der Verteilung des aerodynamischen Feinanteils in einer aus einem Inhalator ausgebrachten Medikamentendosis. Hierzu dient ein (Kaskaden-)Impaktor 75, wobei sich z.B. der "Andersen Kaskadenimpaktor Mark II" mit Stages 0 bis 7 und Filter (ESM Andersen Instruments, Erlangen) eignet. Es handelt sich hierbei um einen mehrstufigen Vieldüsen - Kaskadenimpaktor, welcher sich aus 8 Trennstufen (0, 1, 2, 3, 4, 5, 6, 7, Filter) zusammensetzt. Beispielsweise kann ein bindemittelfreies Borsilikatglasfaserfilter, 76 mm Durchmesser, Porengröße 1 $\mu$m, Rückhaltevermögen $\geq$99.98 %, 0.3 $\mu$m DOP Partikel bei 32 1/min durch 100 cm$^2$, Fa. Pall **Gelman**Sciences 600South Wagner Road.-Ann Arbor, Michigan 481003 im Impaktor eingesetzt werden.

Als Probeneinlass dient ein Sample Induction Port (SIP) 78, z.B. der Andersen-Induction-Port USP (ESM Andersen Instruments, Erlangen). Dieser ist über ein Verbindungsteil "High Top USP" 77 (ESM Andersen Instruments, Erlangen) und einen Präseparator 76 mit dem Impaktor 75 verbunden. Der Präseparator (Vorabscheider) 76 dient zum Abscheiden großer Teilchen. Die Vermessung der Düsendurchmesser der einzelnen Trennstufen wird in regelmäßigen Abständen (mittels Stage Mensuration) überprüft. Neue Impaktoren sind grundsätzlich vor der ersten Benutzung zu vermessen.

Details zu Ausführungsformen des SIP 78 und des High Top USP 77 sind z.B. in der Europäischen Pharmakopoe abgebildet (Eur. Ph. 3, Nachtrag 2000).

In der Abbildung gezeigt ist außerdem eine Impaktor-Standschale 23, die mit der Grundplatte 1 über Befestigungsmittel 47 verankert ist und dem Impaktor 75 festen Halt verleiht.

Saugseitig ist der Impaktor 75 an seinem Fuß über den Verbindungsadapter 81 und weiter über einen Gummi-Vakuumschlauch 40 (D = 8 +/- 0,5 mm, 1 = 450 mm) mit der Steuereinheit verbunden. Da die Grundplatten des Impaktors vom Hersteller mit Kunststoffoliven oder anderen Oliven geliefert werden, die nicht der Forderung der EP/USP von ≥ 8 mm ± 0,5 entsprechen, sind Anpassungen in der beschriebenen Weise erforderlich.

Weiter sind Anschlussteile gezeigt, die am Eingang des SIP 78 anzubringen sind, nämlich der Inhalator-Adapter 72, die Verschlusskappe 73 für den SIP sowie der Adapter 74 mit Olive für den SIP. O-Ringe sind mit dem Bezugszeichen 87 versehen. Der Adapter 74 mit Olive für den SIP dient zur Flusseinstellung und Messung am SIP (siehe Erläuterung weiter unten) und ist mit Hilfe eines Siliconschlauches (Di = 12 x 2,5 mm, L = ca. 50 cm; in Fig. 4 nicht gezeigt) mit dem Flügelradströmungssensor 37 zu verbinden. Über der Inhalator-Adapter 72 für den SIP wird der Inhalator 80 an den SIP 78 so angeschlossen, dass ein spannungsfreier, dichter Sitz gewährleistet ist. Dies ist durch geeignete fertigungstechnische Maßnahmen sicherzustellen. Die Verschlusskappe 73 für den SIP wird verwendet, wenn - vor jeder Messung der aerodynamischen Teilchengröße - der Kaskadenimpaktor 75 auf Dichtigkeit überprüft wird. Hierzu wird der Kaskadenimpaktor 75 mit der Verschlusskappe 73 am SIP 78 verschlossen und ein Druck von etwa 20 - 30 mbar unter dem Normaldruck angelegt. Die Verschlusskappe 73 dient außerdem zum Verschließen des SIP bei der quantitativen Bestimmung des Pulverinhalts nach Erfolg der Ausbringung.

Schließlich ist in Fig. 4 die Verschlusskappe 100 für den High Top USP 77 gezeigt. Mit dieser wird der High Top USP 77 beim Ausspülen zur Bestimmung und quantitativen Ermittlung des darin befindlichen Wirkstoffes verschlossen. Die Verschluss- und Schutzkappe 100 ist innen endseitig mit einem Siliconbelag versehen, durch die der High Top beim Ausspülen durch leichten Druck schonend verschlossen werden kann. Die Kappe 100 dient außerdem zum Schutz der Kanten am Auslass, wenn das Gerät nicht in Gebrauch ist.

Alle übrigen mit Bezugszeichen versehenen Teile wurden bereits zu Fig. 3 erläutert.

Betriebsweise:

Wie oben erläutert, ist die Vorrichtung gemäß der Erfindung geeignet zur Bestimmung der Parameter "abgegebene Dosis" (Messung mit dem Dosis-Sammelrohr) und "Verteilung des aerodynamischen Feinanteils" (Messung mit dem Kaskadenimpaktor). Nachstehend wird die hierzu erforderliche Einstellung der Steuereinheit sowie die Durchführung der Messungen näher beschrieben.

Flusseinstellung mit dem Mess-Sammelrohr:

Die Vakuumpumpe 84, das Steuergerät 89 und die beiden elektronischen Vakuummessgeräte 36 werden angeschaltet. Die Vakuumpumpe 84 wird durch eine Mindestvorlaufzeit von 15 Min. vor Beginn der Flusseinstellung auf die erforderliche Betriebstemperatur gebracht. Das Manometer 22, 29 usw. und die Vakuummessgeräte 36 wurden auf Dichtigkeit geprüft. Diese Dichtigkeitsprüfung ist bei jeder Apparatur Bestandteil der Funktionsprüfung und Wartung. Sie ist grundsätzlich vor dem Betreiben der Steuereinheit durchzuführen.

Ziel der Flusseinstellung ist, den Durchfluss bei am Mess-Sammelrohr 68 angelegtem Vakuum mit dem Durchflusskontrollventil so einzustellen, dass der Druckabfall gemessen an P1 (Fig. 1) innerhalb des Inhalators 80 bzw. mit Ersatzwiderstand 66 4,0 kPa (40,8 cm Wassersäule) beträgt. Gemäß den Vorgaben der EP/USP ist der Druckabfall im Inhalator zu messen. Genauere Angaben enthält die EP/USP nicht. Es hat sich im Falle des "Handi-Haler®" als sinnvoll erwiesen, den Druckabfall im Bereich des Mundstücks zu messen. Bei der Entwicklung einer Prüfmethode ist außerdem ein geeignetes Filter auszuwählen. Dies erfolgt in Abhängigkeit von der jeweiligen Pulvermischung. Das Filter ist so zu wählen, dass kein Durchbruch des Pulvers erfolgt. Beispielsweise hat sich das Glasfaser-Vorfilter GF92 (Schleicher & Schuell AG, 37582 Dassel, Durchmesser 50 mm, Ref.No 10421030) bewährt.

Das Mess-Sammelrohr 68 wird auf dem Halter 10 der Steuereinheit angebracht und saugseitig über einen Adapter 58 und einen Vakuumschlauch 40 mit der Saugeinheit verbunden. Die Pharmakopöen (Pharm. Eur. 2000, USP 24) geben für die Schlauchverbindung 40 die Werte Di = 8 mm ± 0,5 mm, Länge 50 cm ± 10 cm an.

Bei den Kapsel- Pulver- Inhalatoren ist es schwierig, ohne aufwendige Maßnahmen den Fluss in den Inhalatoren zu messen. Außerdem unterliegt der Fluss durch den Inhalator starken Schwankungen, wenn eine Kapsel im Inhalator eingelegt ist. Aus diesen Gründen wird das Mess-Sammelrohr in der Regel mit einem Adapter 67 und eingepasstem Ersatzwiderstand 66 betrieben. Der Ersatzwiderstand 66 muss einen Strömungswiderstand besitzen, der gleich dem des Inhalators 80 mit Kapsel ist, wenn der Strömungswiderstand des Inhalators 80 ohne Kapsel gleich dem Sollwert ist. Ferner kann der Ersatzwiderstand 66 auf der Eintritts- und Austrittsseite leicht mit Strömungsmessgeräten verbunden werden. Der Ersatzwiderstand 66 ist abhängig vom eingesetzten Inhalatortyp und wird nur zur Flusseinstellung eingesetzt. Der Ersatzwiderstand für den "HandiHaler®" ist z. B. so konstruiert, dass er sich über einen Bereich von einem Druck bei ca. 15 bis 50 cm Wassersäule und einem Volumenstrom von ca. 25 bis 45 l/min analog dem "HandiHaler®" mit Kapsel verhält.

Mit einem Vakuumschlauch 38 (Di = 8 mm, Länge 35 cm) wird die Anschlussolive 69 an P1 des Mess-Sammelrohres 68 mit dem als Differenzdruck-Messinstrument zum Einstellen des Druckabfalls in der Saugeinheit integrierten Manometer 22 verbunden. Das Durchfluss-Kontrollventil 33 wird geschlossen, die Steuerung des 2-Wege-Magnetventils 27 auf "Dauer" gestellt. Am Manometer 22, 29 usw. wird der Nullpunkt der Wassersäule eingestellt und das Durchfluss-Kontrollventil 33 vorsichtig geöffnet, bis eine Wassersäule von 40,8 cm eingestellt ist. Der Absolutdruck an beiden Seiten des Durchflusskontrollventils (Messpunkte P2 und P3 - s. Fig. 1) wird abgelesen und dokumentiert. Die einregulierte Stellung des Nadel-Ventils am Durchflusskontrollventil 33 bleibt unverändert. Optional kann eine Sicherung angebracht werden, die den Hahn gegen unbeabsichtigtes Verstellen sichert. Bei angelegter Flussrate muss der Quotient aus dem Druckverhältnis zwischen den Druckmesspunkten P3 zu P2 gleich oder kleiner 0,5 sein ("kritischer Durchfluss"; "critical flow"). Durch Auswechseln des Adapters 67 mit dem eingepassten Ersatzwiderstand 66 gegen einen Adapter 64 zur Flussmessung kann die Durchflussrate mit dem Flügelradströmungssensor 37 gemessen und abgelesen werden (Anzeige am z.B. in das Steuergerät 89 integrierten Messgerät). Dabei ist darauf zu achten, dass der Silicon-Verbindungsschlauch 38 (Länge 50 cm, Di = 12 mm) nicht abgeknickt wird. Die so ermittelte Luftvolumen-Durchflussrate wird als Prüf-Durchflussrate Q in Litern je Minute definiert. Die Prüf-Durchflussrate Q hat eine Toleranz von ± 5%. Die Prüf-Durchflussdauer t in Sekunden wird so festgelegt, dass ein bestimmtes Volumen von beispielsweise 4 Litern durch den Inhalator 80 strömt. Die genannten 41 werden von der EP/USP gefordert. Durch entsprechende Umrechnung der Saugzeit können mit der erfindungsgemäßen Steuereinheit auch weitere Volumina problemlos umgesetzt werden.

Beispiel zur Berechnung der Saugzeit:

$$\text{Saugzeit} = \frac{60 \text{ Sekunden x gefordertes Saugvolumen}}{\text{abgelesener Durchfluss}}$$

Bei einem Fluss von beispielsweise 391/min ergibt sich eine Saugzeit von 6,15 sec. Bei abweichenden Prüf-Durchflussraten ergeben sich andere Saugzeiten zur Erreichung eines geforderten Luftvolumens von 4 Litern.

Vor der eigentlichen Bestimmung der ausgebrachten Dosis wird das Mess-Sammelrohr 68 gegen ein Dosis-Sammelrohr 60 ausgewechselt. Durch das Auswechseln des HandiHaler®-Adapters 62 gegen den "Adapter zur Flussmessung" 64 und Anschluss an den Flügelradströmungssensor 37 kann der angelegte Fluss zur Kontrolle gemessen werden. Die Steuerung des 2-Wege-Magnetventils 27 wird am Steuergerät 89 auf "Timer" gestellt und an der Zeiteinstellung (Timer) die errechnete Saugzeit eingestellt.

Die Steuereinheit wird also einerseits mit dem Mess-Sammelrohr und andererseits mit einem Dosis-Sammelrohr betrieben. Das Mess-Sammelrohr dient nur zur Einstellung und Ermittlung der erforderlichen Messparameter, das Dosissammelrohr hingegen zur eigentlichen Bestimmung der ausgebrachten Dosis. Grund hierfür ist die in der Praxis erforderliche **quantitative** Mehrfachbestimmung. Hierzu ist ein dichtes und leicht "reinigbares" System erforderlich. Beim Messsammelrohr ist beispielsweise bedingt durch den P1-Stutzen reinigungsmäßig und auch meßtechnisch ein gewisses Gefahrenpotential dergestalt gegeben, dass sich dort Pulver einlagern kann. Dies gilt besonders bei Bestimmungen im spurenanalytischen Bereich.

Sollte mit einem Inhalator eingestellt werden, für den es keinen Ersatzwiderstand gibt, so muss der jeweilige Inhalator in der entsprechenden Betriebsweise mit einem entsprechenden Widerstand berücksichtigt und eingebaut werden.

Ausbringung des Wirkstoffs und Bestimmung der abgegebenen Dosis:

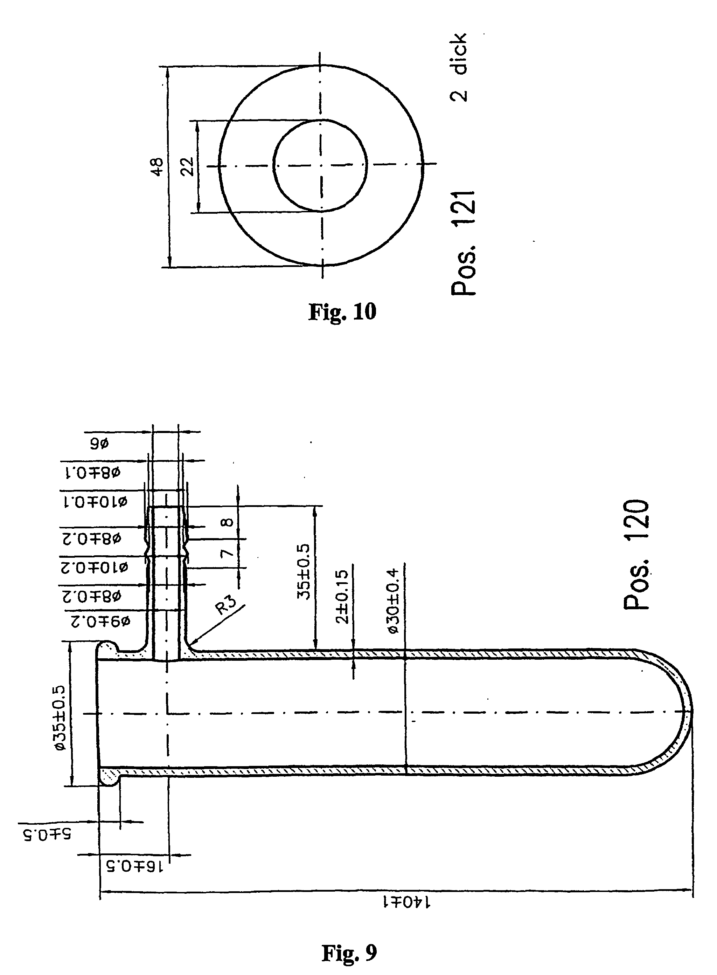

Die Bestimmung der Ausbringung wird mit einem Dosis-Sammelrohr durchgeführt. Sind alle Betriebsparameter entsprechend den geltenden Prüfungsvorschriften an der Steuereinheit eingestellt, die Anlage entsprechend überprüft, der Inhalator 80 (beispielsweise der HandiHaler®) qualifiziert und der erforderliche Fluss eingestellt, kann mit der Bestimmung der Ausbringung begonnen werden. Der Inhalator wird mit einer Kapsel beschickt, die Kapsel gelocht und der geschlossene Inhalator in den Adapter 62 im Dosis-Sammelrohr 60 eingesetzt. Dann wird der Startknopf gedrückt, wobei durch die eingestellte Zeitsteuerung das Magnetventil 27 geöffnet und der Inhalt der Kapsel durch einen Entleerungsvorgang ausgebracht wird. Dabei ist darauf zu achten, dass der Quotient der Drücke P3/P2 kleiner 0,5 ist. Ausbringungen aus weiteren Kapseln erfolgen in der Prüfungsvorschrift angegebenen Anzahl. Der Inhalator 80 wird nach jeder Ausbringung abgenommen und der Inhalt entsprechend der Prüfungsvorschrift für die Bestimmung aufgearbeitet. Jede Kapsel wird in einem separaten Dosis-Sammelrohr 60 ausgebracht und entsprechend aufgearbeitet. Der in der Regel extrahierte Inhalt des Dosis-Sammelrohres 60 wird in einen Saugfinger 120 (Fig. 9) durch kurzzeitiges Anlegen eines Unterdruckes abgesaugt. Zum Absaugen wird der Dichtring 121 (Fig. 10) zwischen Saugfinger 120 und Dosis-Sammelrohr angebracht. Die quantitative Bestimmung erfolgt in der Regel mittels HPLC.

Bestimmung der Verteilung des aerodynamischen Feinanteils:

Flusseinstellung am Kaskadenimpaktor:

Der zusammengebaute Kaskadenimpaktor 75 wird aufrecht stehend in die an der Steuereinheit dafür vorgesehenen Schale 23 positioniert und mit dem SIP 78 verbunden. Vor jeder Bestimmung ist eine Dichtigkeitsprüfung am Impaktor durchzuführen. Der SIP 78 wird mit der Verschlusskappe 73 verschlossen. Bei laufender Vakuumpumpe 84, eingeschaltetem Steuergerät 89 (Magnet-Ventil dauerhaft geöffnet) und Vakuummessgeräten 36, sowie geschlossenem Durchflusskontrollventil 33 wird vorsichtig ein Unterdruck von etwa 20 bis 30 mbar durch Öffnen des Durchflusskontrollventils 33 eingestellt. Das Vakuummessgerät 36 am Messpunkt P2 (rechtes Manometer) wird bei geschlossenem Nadelventil beobachtet. Der eingestellte Unterdruck darf maximal 1 mbar/ sec abnehmen.

Anschließend wird am Eingang des SIP 78 über einen Anschlussadapter 74 mit Olive für SIP der Flügelradströmungssensor 37 angeschlossen und der im Rahmen der Bestimmung der abgegebenen Dosis ermittelte und definierte Volumenfluss von beispielsweise 39 Liter/min eingestellt. Damit wird zugleich gewährleistet, dass beide Bestimmungen, also die "Gleichförmigkeit der ausgebrachten Dosis" und die "Verteilung des aerodynamischen Feinanteils", bei gleichem Fluss durchgeführt werden. Liegt eine Vorgabe zum einzustellenden Volumenfluss nicht vor, so kann er wie oben beschrieben mit den entsprechenden Devices in Verbindung mit dem Mess-Sammelrohr ermittelt werden. Um das gewünschte Saugvolumen von beispielsweise 4 Litern zu erzielen, wird am Timer des Steuergerätes 89, die aus dem Volumenfluss errechnete Saugzeit von 6,15 Sekunden eingestellt. Bei angelegter Flussrate muss der kritische Durchfluss (Quotient P3/P2 gleich oder kleiner 0,5) gewährleistet sein.

Zum Einbringen des Kapselinhaltes in den Impaktor 75 wird ein geeigneter Inhalator-Adapter 72 mit vorbereitetem Inhalator 80, wie bei der Ausbringung beschrieben, auf den SIP 78 aufgesetzt. Das Steuergerät wird zuvor auf Timer-Steuerung gestellt. Das Öffnen des Magnetventiles 27 erfolgt durch Starten des Timers an dem elektronischen Steuergerät 89. Das Pulver wird in den Impaktor 75 ausgebracht. Der Impaktor 75 wird anschließend zerlegt, die Rückstände beispielweise im Einlassteil (Adapter 72, Sample Induction Port 75, High Top USP 77), im Präseparator 76, auf den Prallplatten der Kaskaden 0 bis 7 und dem Filter aufgearbeitet und bestimmt.

Die Erfindung ist nicht auf die in den Abbildungen gezeigten Ausführungsformen beschränkt, sondern umfasst im Rahmen der Ansprüche z.B. auch Ausführungsformen, in denen fachnotorisch austauschbare Mittel verwendet werden. Diese sind dem Fachmann bekannt.

**Patentansprüche**

1. Steuereinheit zur Flussregulierung zum Einsatz in einer Vorrichtung zur Prüfung der Ausbringung einer Pulverdosis aus einem Pulverinhalator, enthaltend

   - ein durch eine Schaltuhr steuerbares erstes Ventil,
   - eine Schaltuhr zur Steuerung des ersten Ventils,
   - ein mit dem ersten Ventil verbundenes, die Luftdurchleitung ermöglichendes Verbindungsstück,
   - ein an das Verbindungsstück anschließendes Durchflusskontrollventil,
   - einen am ersten Ventil vorgesehenen Anschluss für eine Saugvorrichtung,
   - einen am Durchflusskontrollventil vorgesehenen Anschluss für ein Sammelrohr oder einen Impaktor und
   - ein Mittel zur Messung des Luftvolumenstromes durch das Sammelrohr, bzw. den Impaktor

   wobei das Mittel zur Messung des Luftvolumenstromes ein kalibrierbarer Strömungssensor ist.

2. Steuereinheit nach Anspruch 1, bei der der kalibrierbare Strömungssensor ein Flügelradströmungssensor ist.

3. Steuereinheit nach Anspruch 1 oder 2, bei der an einem an den Strömungssensor angeformten Ansaugrohr ein Dämpfungsfilter angebracht ist.

4. Steuereinheit nach einem der Ansprüche 1 bis 3, bei der die Schaltuhr und eine Vorrichtung zur Anzeige der mit dem kalibrierbaren Strömungssensor gemessenen Werte in einem Steuergerät integriert sind.

5. Steuereinheit zur Flussregulierung zum Einsatz in einer Vorrichtung zur Prüfung der Ausbringung einer Pulverdosis aus einem Pulverinhalator, enthaltend

   - ein durch eine Schaltuhr steuerbares erstes Ventil,
   - eine Schaltuhr zur Steuerung des ersten Ventils,
   - ein mit dem ersten Ventil verbundenes, die Luftdurchleitung ermöglichendes Verbindungsstück,
   - ein an das Verbindungsstück anschließendes Durchflusskontrollventil,
   - einen am ersten Ventil vorgesehenen Anschluss für eine Saugvorrichtung,

- einen am Durchflusskontrollventil vorgesehenen Anschluss für ein Sammelrohr oder einen Impaktor,
- zwei in Saugrichtung vor und nach dem Durchflusskontrollventil angebrachten Anschlussstellen für jeweils ein Druckmessgerät,
- über jeweils ein Verbindungsstück an den Anschlussstellen angebrachte Druckmessgeräte,

wobei die Verbindungsstücke aus einer inneren, die Luftleitung ermöglichenden Kapillare und einem die Kapillare ummantelndem Außenrohr bestehen.

6. Sammelrohr in Verbindung mit einer Steuereinheit gemäß einem der Ansprüche 1 bis 5, wobei die innere Oberfläche des Sammelrohres mit einer Rauhtiefe von höchstens 6,3 μm eine sehr glatte Fläche aufweist.

7. Sammelrohr nach Anspruch 6, in Verbindung mit einer Steuereinheit gemäß einem der Ansprüche 1 bis 5, das die Grundform eines Hohlzylinders aufweist, an einer Seite zwei Einkerbungen zur Aufnahme je eines O-Rings aufweist und an der gegenüberliegenden Seite des Hohlzylinders konisch geformt ist.

8. Vorrichtung zur Prüfung der Ausbringung einer Pulverdosis aus einem Pulverinhalator, enthaltend

   - eine Steuereinheit zur Flussregulierung gemäß einem der Ansprüche 1 bis 5,
   - eine am Anschluss für eine Saugvorrichtung angebrachte Vakuumpumpe und
   - ein Sammelrohr gemäß einem der Ansprüche 6 oder 7 oder einen Kaskadenimpaktor.

9. Verfahren zur Prüfung der Ausbringung einer Pulverdosis aus einem Pulverinhalator unter Verwendung einer Vorrichtung gemäß Anspruch 8, wobei in einem ersten Schritt unter Verwendung eines Mess-Sammelrohres die zur Erreichung des geforderten Saugvolumens erforderliche Saugzeit ermittelt wird und in einem zweiten Schritt unter Verwendung eines Dosis-Sammelrohres in der ermittelten Saugzeit Pulver aus einem am Dosis-Sammelrohr angebrachten Pulverinhalator in das Dosis-Sammelrohr eingebracht wird, wobei das Dosis-Sammelrohr im Gegensatz zum Mess-Sammelrohr keine Anschlussolive zum Anschluss eines Druckmessgerätes aufweist.

**Claims**

1. Control unit for flow regulation for use in an apparatus for testing the delivery of a dose of powder from a powder inhaler, containing

   - a first valve controllable by a time switch,
   - a time switch for controlling the first valve,
   - a connector connected to the first valve and allowing air to pass through,
   - a throughflow control valve connected to the connector,
   - a connector for a suction device provided on the first valve,
   - a connector for a collecting tube or an impactor, provided on the throughflow control valve, and
   - a means for measuring the airflow volume through the collecting tube or the impactor,

   wherein the means for measuring the airflow volume comprises a calibratable flow sensor.

2. Control unit according to claim 1, wherein the calibratable flow sensor is a flywheel flow sensor.

3. Control unit according to claim 1 or 2, wherein a damping filter is mounted on an intake tube formed on the flow sensor.

4. Control unit according to one of claims 1 to 3, wherein the time switch and an apparatus for indicating the values measured by the calibratable flow sensor are integrated in a control device.

5. Control unit for flow regulation for use in an apparatus for testing the delivery of a dose of powder from a powder inhaler, containing

   - a first valve controllable by a time switch,
   - a time switch for controlling the first valve,
   - a connector connected to the first valve and allowing air to pass through,
   - a throughflow control valve connected to the connector,
   - a connector for a suction device provided on the first valve,
   - a connector for a collecting tube or an impactor, provided on the throughflow control valve, and
   - means for measuring the airflow volume through the collecting tube or an impactor,
   - two connecting points for a pressure gauge provided in front of and behind the throughflow control valve in the direction of suction,
   - pressure measuring gauges each mounted at the connecting point via a connecting member,

   wherein the connecting members consist of an inner capillary which carries the air and an outer tube which encases the capillary.

6. Collecting tube in conjunction with a control unit according to one of claims 1 to 5, wherein the inner surface of the collecting tube with a peak-to-valley height of at most 6.3 μm has a very

smooth surface.

7. Collecting tube according to claim 6 in conjunction with a control unit according to one of claims 1 to 5 , that in the basic shape of a hollow cylinder having two notches each for receiving an O-ring on one side and conically formed on the other side of the hollow cylinder.

8. Apparatus for testing the delivery of a dose of powder from a powder inhaler, containing

   - a control unit for flow regulation according to one of claims 1 to 5,
   - a vacuum pump mounted on the connection for a suction device and
   - a collecting tube according to one of claims 6 or 7 or a cascade impactor.

9. Process for testing the delivery of a dose of powder from a powder inhaler using an apparatus according to claim 8, wherein in a first step using a measuring collecting tube the suction time needed to achieve the required suction volume is determined and in a second step using a dose collecting tube in the suction time determined powder is introduced into the dose collecting tube from a powder inhaler mounted on the dose collecting tube, the dose collecting tube, unlike the measuring collecting tube, having no connecting olive for the attachment of a pressure gauge.

**Revendications**

1. Unité de commande pour une régulation de flux pour une utilisation dans un dispositif servant au contrôle de l'extraction d'une dose de poudre hors d'un inhalateur de poudre comprenant :

   - une première vanne pouvant être commandée par une horloge de commutation,
   - une horloge de commutation pour la commande de la première vanne,
   - une pièce de liaison reliée à la première vanne et permettant de guider le passage de l'air,
   - une vanne de contrôle du flux faisant suite à la pièce de liaison,
   - un raccord prévu sur la première vanne, pour un dispositif d'aspiration
   - un raccord prévu sur la vanne de contrôle du flux, pour un tube collecteur ou pour un impacteur et
   - un moyen pour la mesure du flux volumique de l'air à travers le tube collecteur ou l'impacteur

   dans laquelle le moyen pour la mesure du flux volumique de l'air est un capteur de flux pouvant être calibré.

2. Unité de commande selon la revendication 1, dans laquelle le capteur de flux pouvant être calibré est un capteur de flux à roue à ailettes.

3. Unité de commande selon la revendication 1 ou 2, dans laquelle, sur un tuyau d'aspiration formé sur le capteur de flux, est disposé un filtre d'atténuation.

4. Unité de commande selon l'une quelconque des revendications 1 à 3, dans laquelle l'horloge de commutation et un dispositif pour l'affichage des valeurs mesurées avec le capteur de flux pouvant être calibré sont intégrés dans un équipement de commande.

5. Unité de commande pour une régulation de flux pour une utilisation dans un dispositif servant au contrôle de l'extraction d'une dose de poudre hors d'un inhalateur de poudre comprenant :

   - une première vanne pouvant être commandée par une horloge de commutation,
   - une horloge de commutation pour la commande de la première vanne,
   - une pièce de liaison reliée à la première vanne et permettant de guider le passage de l'air,
   - une vanne de contrôle du flux faisant suite à la pièce de liaison,
   - un raccord prévu sur la première vanne, pour un dispositif d'aspiration
   - un raccord prévu sur la vanne de contrôle du flux, pour un tube collecteur ou pour un impacteur
   - deux points de raccordement pour respectivement un équipement de mesure de la pression disposés, en direction d'aspiration, en amont et en aval de la vanne de contrôle du flux, et
   - des équipements de mesure de la pression disposés aux points de raccordement par l'intermédiaire de respectivement une pièce de liaison,

   dans laquelle les pièces de liaison se composent d'un tube capillaire interne permettant le guidage de l'air et d'un tube extérieur entourant le tube capillaire.

6. Tube collecteur en liaison avec une unité de commande selon l'une quelconque des revendications 1 à 5, dans lequel la surface intérieure du tube collecteur présente, avec une profondeur d'aspérité de rugosité d'au maximum 6,3 μm, une surface très lisse.

7. Tube collecteur selon la revendication 6 en liaison avec une unité de commande selon l'une quelconque des revendications 1 à 5, qui a la forme de base d'un cylindre creux, et comprend sur un côté deux entailles pour recevoir respectivement un joint torique et qui a une forme conique sur le côté opposé du cylindre creux.

8. Dispositif pour le contrôle de l'extraction d'une dose de poudre hors d'un inhalateur de poudre comprenant :

   - une unité de commande pour la régulation du flux selon l'une quelconque des revendications 1 à 5,
   - une pompe à vide disposée sur le raccord pour un dispositif d'aspiration et
   - un tube collecteur selon l'une quelconque des revendications 6 ou 7 ou un impacteur en cascade.

9. Procédé pour le contrôle de l'extraction d'une dose de poudre hors d'un inhalateur de poudre en utilisant un dispositif selon la revendication 8, dans lequel, lors d'une première étape, le temps d'aspiration nécessaire pour obtenir le volume d'aspiration requis est déterminé en utilisant un tube collecteur de mesure, et, lors d'une deuxième étape, en utilisant un tube collecteur de doses, de la poudre est, pendant le temps d'aspiration déterminé, introduite dans le tube collecteur de doses à partir d'un inhalateur à poudre disposé sur le tube collecteur de doses, le tube collecteur de doses ne comprenant, contrairement au tube collecteur de mesure, pas d'olive de raccordement pour le branchement d'un équipement de mesure de la pression.

Fig. 1

- Eingang
- Sammelrohr — A
- P1
- Mundstück-adapter
- Filter — B
- Vakuumschlauch — D
- P2
- Durchfluß-Kontroll-ventil — H
- Verbindungs-stück — C
- P3
- Schaltuhr — G
- 2-Wege-Magnet-ventil — E
- Vakuum-pumpe — F

**E**

Schaltuhr

**D**

Vakuumpumpe

P3    P2

Impaktor

2-Wege-
Magnetventil
**C**

Durchfluß-
kontrollventil
**F**

Verbindungsstück **A**

Verbindungsschlauch **B**

**Fig. 2**

Fig. 3

**Fig. 4**

**Fig. 5**

18

47

⌀4

⌀2.2

⌀11.5

65

Pos. 19 angelötet

60°

R40

20

101

150

Rohr und Kapilare mit
Pos. 17 bzw. Pos. 18 verlöten

⌀13.5

2

A

B

Fig. 6

Pos. 72

ø37
ø30
ø24.8
M4
3
6
2
2
2
2.5
7 +1
30
20
14
5
ø38.2+0.1
ø41.4-0.1
ø60

mit Silikon
Kautschuk ausgiesen

40
20
22.5°
45°
ø5
1.5

Einguß Handi Haler

Fig. 7

**Fig. 8**

**Fig. 10**

Pos. 121

2 dick

**Fig. 9**

Pos. 120